# EUROPEAN PATENT APPLICATION

(11) **EP 4 729 543 A1**
(43) Date of publication of application: **22.04.2026**
(21) Application number: 24207379.9
(22) Date of filing: 18.10.2024
(51) Int. Cl.: C07K 16/28, A61K 39/395

(54) **ANTI-IL-4 RECEPTOR ANTIBODIES FOR VETERINARY USE**

(71) Applicant: Harfang Therapeutics, 56000 Vannes (FR)
(72) Inventor: Guillo, Maël, 85370 Corpe (FR); TILLET, Yves, 77400 LAGNY-SUR-MARNE (FR)
(74) Representative: Alatis

(57) **Abstract**

The present invention relates to antibodies, or antigen-binding fragments thereof, that bind to canine, feline and/or equine alpha subunit of the IL-4/IL-13 receptor (IL-4R), and pharmaceutical compositions comprising such antibodies or fragments. The invention also relates to methods of using the same, for example method of treating a disease or disorder associated with IL-4R dysfunction in a canine, feline or equine species in need thereof.

## Description

### FIELD OF THE INVENTION

This invention relates to antibodies, or antigen-binding fragments thereof, that bind to canine, feline and/or equine alpha subunit of the IL-4/IL-13 receptor (IL-4R), and pharmaceutical compositions comprising such antibodies or fragments. The invention also relates to methods of using the same, for example method of treating a disease or disorder associated with IL-4R dysfunction in a canine, feline or equine species in need thereof.

### BACKGROUND

Allergic diseases like asthma and atopic dermatitis are driven by type 2 inflammation, characterized by elevated levels of cytokines such as IL-4, IL-13, and IL-5. These cytokines activate immune cells and promote IgE production. Biologics targeting these cytokines or their receptors have shown promise, with IL-4/IL-13 signaling being a key target due to its role in TH2 cell differentiation and downstream inflammation.

IL-4/IL-13 signaling represents a strategic approach for inhibiting type 2 inflammation and treating atopic dermatitis in companion animals.

IL-4 can signal through two types of heterodimeric receptor complexes: the Type I receptor which is composed of IL-4 receptor alpha subunit (IL-4Rα) and cytokine receptor common γ-chain (yc), and the Type II receptor which is composed of IL-4Rα and the IL-13 receptor alpha 1 subunit (IL-13Rα1). In contrast, IL-13 signals exclusively through the Type II receptor. Therefore, targeting IL-4Rα (also called IL-4R) provides a means to inhibit signaling from both IL-4 and IL-13 cytokines. Therefore, antibodies that target IL-4R offer a strategic approach to block the signaling pathways of both IL-4 and IL-13.

Regarding the treatment of atopic dermatitis in humans, monoclonal antibodies directed against human IL-4R, i.e., Dupilumab (Dupixent^{™}), a humanized IgG4 Ab, have been approved by U.S. Food & Drug Administration for this purpose.

Regarding companion animals, the development of IL-4R-targeting antibodies for canine species is an active area of research. Caninized antibodies to canine IL-4Rα have been described in the prior art, for example in WO 2021/188631 A1 (Kindred Biosciences, Inc.) which describes isolated anti-IL-4 receptor (or IL-4R) antibodies binding to canine or feline IL-4R. Additionally, WO 2021/123089 A1 (Intervet Inc.) describes antibodies to canine IL-4R that can block the binding of canine IL-4 and/or IL-13.

Monoclonal antibodies have been used in human medicine for thirty years; however, their use in veterinary medicine has historically been limited. In July 2017, the European Medicines Agency (EMA) made a significant advancement by granting the first marketing authorization for a caninized monoclonal antibody, lokivetmab (Cytopoint^{®}). These antibodies are used in dogs to alleviate symptoms associated with atopic dermatitis. However, these antibodies target anti-interleukin 31 (IL-31) which targets pruritus, treating one of the symptoms of atopic dermatitis only, without preventing the inflammatory reaction from occurring with its consequences: alterations in the appearance of the skin and skin superinfections by bacteria and fungi which take advantage of this inflammatory state to settle in. The EMA also approved bedinvetmab (Librela^{®}) as a monoclonal antibody specifically designed to address osteoarthritis in dogs. These antibodies target and neutralize nerve growth factor (NGF), a key player in the pain signaling pathway. By inhibiting NGF, they provide effective pain relief for dogs suffering from osteoarthritis, thereby improving their overall quality of life.

Despite recent publications, current biotherapies for atopic dermatitis in canine species do not offer efficient relief from itching, nor do they achieve significant effects on skin inflammation or improvement in skin barrier function. Therefore, there is a need to develop more effective biotreatments that address at least one of these symptoms, and ideally all of them.

One of the objects of the present invention is to provide antibodies that bind to canine, feline and/or equine alpha subunit of the IL-4/IL-13 receptor (IL-4R) with high affinity.

Another object of the invention is to provide biotherapies (herein, therapies based on administering antibodies), for canine, feline or equine species, that address both pruritus and inflammation, rather than targeting only one of these conditions.

Another object of the invention is to provide antibodies with reduced immunogenicity, improved interaction with canine immune cells and with a higher degree of specificity and affinity for canine antigens.

Another object of the invention is to offer a veterinary therapy to treat canine species in accordance with animal welfare.

### BRIEF SUMMARY

A first object of the present invention relates to an isolated antibody, or antigen-binding fragment thereof, that binds to canine, feline and/or equine alpha subunit of the IL-4/IL-13 receptor (IL-4R), wherein the antibody or fragment comprises one of the following complementary determining region (CDR) sets:
a) heavy chain CDRs 1-3 (CDRH1-3) comprising the amino acid sequences of SEQ ID NO: 1, 4 and 7, respectively, and light chain CDRs (CDRL1-3) comprising the amino acid sequences of SEQ ID NO: 10, 13 and 16, respectively;
b) heavy chain CDRs 1-3 (CDRH1-3) comprising the amino acid sequences of SEQ ID NO: 2, 5 and 8, respectively, and light chain CDRs (CDRL1-3) comprising the amino acid sequences of SEQ ID NO: 11, 14 and 17, respectively; or
c) heavy chain CDRs 1-3 (CDRH1-3) comprising the amino acid sequences of SEQ ID NO: 3, 6 and 9, respectively, and light chain CDRs (CDRL1-3) comprising the amino acid sequences of SEQ ID NO: 12, 15 and 18, respectively.

Another object of the present invention relates to an isolated antibody, or antigen-binding fragment thereof, that binds to canine, feline and/or equine alpha subunit of the IL-4/IL-13 receptor (IL-4Rα), wherein the antibody or fragment comprises one of the following heavy chain (VH) and light chain (VL) sets:
a) a VH comprising an amino acid sequence at least 90%, at least 95% identical or 100% identical to SEQ ID NO: 19 and a VL comprising an amino acid sequence at least 90%, at least 95% identical or 100% identical to SEQ ID NO: 22;
b) a VH comprising an amino acid sequence at least 90%, at least 95% identical or 100% identical to SEQ ID NO: 20 and a VL comprising an amino acid sequence at least 90%, at least 95% identical or 100% identical to SEQ ID NO: 23; or
c) a VH comprising an amino acid sequence at least 90%, at least 95% identical or 100% identical to SEQ ID NO: 21 and a VL comprising an amino acid sequence at least 90%, at least 95% identical or 100% identical to SEQ ID NO: 24.

The antibody or fragment of the present invention is preferably a fully canine antibody.

Another object of the present invention relates to an isolated nucleic acid that encodes the antibody, or antigen-binding fragment thereof, of the present invention.

Another object of the present invention relates to an expression vector comprising the nucleic acid of the present invention.

Another object of the present invention relates and isolated host cell comprising the nucleic acid of the present invention or the expression vector of the present invention, or that expresses the antibody or antigen-binding fragment thereof of the present invention.

Another object of the present invention relates to a pharmaceutical composition comprising the antibody of the present invention and a pharmaceutically acceptable carrier, or comprising the nucleic acid of the present invention and a pharmaceutically acceptable carrier.

Another object of the present invention relates to a method of producing an antibody or antigen-binding fragment thereof that binds to IL-4R, comprising incubating the host cell of the present invention under conditions suitable for producing the antibody or antigen-binding fragment thereof.

Another object of the present invention relates to a method of treating a disease or disorder associated with IL-4R dysfunction in a canine, feline or equine species in need thereof, the method comprising administering to the canine, feline or equine species a therapeutically effective amount of the antibody or antigen-binding fragment thereof of the present invention or the pharmaceutical composition of the present invention.

The disease or disorder may be a chronic inflammatory skin condition; preferably selected in the group consisting of atopic dermatitis, allergic dermatitis, flea allergy dermatitis, contact dermatitis, asthma, seborrhea, pruritus, psoriasis, scleroderma, eczema and pyoderma, and other hypersensitivity reactions involving the IL-4/IL-13R receptor; more preferably, atopic dermatitis.

### DETAILED DESCRIPTION

The present disclosure employs unless otherwise indicated, conventional molecular biology techniques, which are within the skill of the art. Unless defined otherwise, all technical and scientific terms used herein have the same meaning as is commonly understood by one of ordinary skill in the art.

### Definitions

Unless specifically stated or obvious from context, as used herein, the term "about" in reference to a number or range of numbers is understood to mean the stated number and numbers +/- 10% thereof, or 10% below the lower listed limit and 10% above the higher listed limit for the values listed for a range.

The term "antibody" as used herein encompasses various antibody structures, including but not limited to monoclonal antibodies, polyclonal antibodies, multispecific antibodies (for example, bispecific (such as Bi-specific T-cell engagers) and trispecific antibodies), and various antibody fragments (such as Fab, F(ab')2, ScFv, minibody, diabody, triabody, and tetrabody) provided they exhibit the desired antigen-binding activity.

The term "monoclonal antibody" as used herein refers to an antibody derived from a substantially homogeneous population of antibodies, meaning that the individual antibodies in the population are identical, except for possible minor naturally occurring mutations. Monoclonal antibodies are highly specific, targeting a single antigenic site. Unlike a population of "polyclonal antibodies" which consists of a mixture of antibodies directed against different epitopes, each monoclonal antibody recognizes and binds to a single epitope on the antigen. The term "monoclonal" denotes that the antibody originates from a homogeneous population of identical antibodies and does not imply any specific production method. Monoclonal antibodies can be produced using various techniques, including the hybridoma method as described by Kohler and Milstein (1975, Nature 256:495), recombinant DNA methods (e.g., U.S. Pat. No. 4,816,567), or isolated from phage libraries as outlined by McCafferty, et al., (1990, Nature 348:552-554).

The term "canine antibody" as used herein refers to an antibody that is 100% derived from canine immune systems. It is fully composed of sequences that are naturally found in canine species, without any genetic modification or sequence derived from another species. The term "canine antibodies" is therefore opposed to the term "caninized antibodies" or "chimeric antibodies" which denote antibodies that incorporate some elements from canine species, such as the constant regions or specific framework regions, while retaining other components (e.g., CDRs) from different species, such as human or mouse.

The term "binding" as used herein refers to the interaction, association, or affinity of an antibody with a specific antigen or epitope. This binding can be assessed using various established methods known in the art, such as surface plasmon resonance (SPR), biolayer interferometry (BLI), immunoblotting, enzyme-linked immunosorbent assay (ELISA), or KinExA. An antibody is said to exhibit "binding" if it can be detected through these techniques.

The term "nucleic acid" as used herein encompasses double- or triple-stranded nucleic acids, as well as single-stranded molecules. In double- or triple-stranded nucleic acids, the nucleic acid strands need not be coextensive (i.e., a double-stranded nucleic acid need not be double-stranded along the entire length of both strands). Nucleic acid sequences, when provided, are listed in the 5' to 3' direction, unless stated otherwise. A "nucleic acid" as referred to herein can comprise at least 5, 10, 20, 30, 40, 50, 60, 70, 80, 90, 100, 125, 150, 175, 200, 225, 250, 275, 300, 325, 350, 375, 400, 425, 450, 475, 500, 600, 700, 800, 900, 1000, or more bases in length. The following are non-limiting examples of polynucleotides: coding or non-coding regions of a gene or gene fragment, intergenic DNA, loci (locus) defined from linkage analysis, exons, introns, messenger RNA (mRNA), transfer RNA, ribosomal RNA, short interfering RNA (siRNA), short-hairpin RNA (shRNA), micro-RNA (miRNA), small nucleolar RNA, ribozymes, complementary DNA (cDNA), which is a DNA representation of mRNA, usually obtained by reverse transcription of messenger RNA (mRNA) or by amplification; DNA molecules produced synthetically or by amplification, genomic DNA, recombinant polynucleotides, branched polynucleotides, plasmids, vectors, isolated DNA of any sequence, isolated RNA of any sequence, nucleic acid probes, and primers. cDNA encoding for a gene or gene fragment referred herein may comprise at least one region encoding for exon sequences without an intervening intron sequence in the genomic equivalent sequence.

The terms "polypeptide" and "protein" are used interchangeably to refer to polymers of amino acid residues, without a minimum length restriction. These polymers may include natural or non-natural amino acid residues and encompass a range of structures such as peptides, oligopeptides, dimers, trimers, and multimers. The definition includes both full-length proteins and their fragments. Additionally, the terms cover post-translational modifications of the polypeptide, such as glycosylation, sialylation, acetylation, and phosphorylation. For the purposes of this disclosure, a "polypeptide" also includes proteins with modifications, including deletions, additions, or substitutions (typically conservative), provided they retain the desired activity. These modifications can be intentional, such as those introduced by site-directed mutagenesis, or incidental, resulting from natural mutations in host organisms or errors during PCR amplification.

The term "sequence identity" refers to the exact match of two polynucleotide sequences, compared nucleotide-by-nucleotide, over a specified window of comparison. The "percentage of sequence identity" is calculated by comparing two optimally aligned sequences within this window, counting the number of positions where identical nucleic acid bases (e.g., A, T, C, G, U, or I) are present in both sequences, dividing the number of matched positions by the total number of positions in the comparison window (the window size), and multiplying the result by 100 to obtain the percentage of sequence identity.

The term "homology" or "similarity" between two proteins refers to the comparison of their amino acid sequences, including conserved amino acid substitutions, between one protein sequence and another. The replacement of one amino acid in a polypeptide with another term may be called "amino acid substitution". This substitution may be conservative, where the substituted amino acid shares similar properties with the original; for example, replacing Ala (A) with Val (V), Leu (L) or Ile (I) represents conservative substitutions because these amino acids share similar properties. Amino acid substitution may be introduced to achieve desired activities, such as enhanced antigen binding, reduced immunogenicity, improved antibody-dependent cellular cytotoxicity or complement-dependent cytotoxicity, better recombinant production, and/or improved pharmacokinetics, while maintaining a high level of homology with the original protein sequence. Amino acids can be classified according to common side chain properties.
(1) Hydrophobic: Norleucine, Met, Ala, Val, Leu, Ile;
(2) neutral hydrophilicity: Cys, Ser, Thr, Asn, Gln;
(3) acidic: Asp, Glu;
(4) basic: His, Lys, Arg;
(5) residues that affect chain orientation: Gly, Pro;
(6) Aromatics: Trp, Tyr, Phe.

Identity and homology/similarity may be determined using, for example, the BLAST (Basic Local Alignment Search Tool) program available through the National Center for Biological Information (NCBI).

The term "affinity" as used herein refers to the overall strength of non-covalent interactions between a single binding site of the antibody and its antigen. The affinity of molecule X for its partner Y is commonly expressed as a dissociation constant (Kd). Affinity can be measured using methods known in the art, such as surface plasmon resonance (SPR), biolayer interferometry (BLI), enzyme-linked immunosorbent assay (ELISA), immunoblotting, or KinExA.

Antibodies that bind canine IL-4R, feline IL-4R and/or equine IL-4R are provided. Antibody heavy chains and light chains that are capable of forming antibodies that bind IL-4R are also provided. In addition, antibodies, heavy chains, and light chains comprising one or more complementary determining regions (CDRs) are provided. Polynucleotides encoding antibodies to canine, feline or equine IL-4R are provided. Methods of producing or purifying antibodies to canine, feline or equine IL-4R are also provided. Methods of treatment using antibodies to canine, feline or equine IL-4/IL-13 are provided.

### Antibodies

The present invention relates to an isolated antibody, or antigen-binding fragment thereof, wherein the antibody or fragment comprises one of the following complementary determining region (CDR) sets:
a) heavy chain CDRs 1-3 (CDRH1-3) comprising the amino acid sequences of SEQ ID NO: 1, 4 and 7, respectively, and light chain CDRs (CDRL1-3) comprising the amino acid sequences of SEQ ID NO: 10, 13 and 16, respectively;
b) heavy chain CDRs 1-3 (CDRH1-3) comprising the amino acid sequences of SEQ ID NO: 2, 5 and 8, respectively, and light chain CDRs (CDRL1-3) comprising the amino acid sequences of SEQ ID NO: 11, 14 and 17, respectively; or
c) heavy chain CDRs 1-3 (CDRH1-3) comprising the amino acid sequences of SEQ ID NO: 3, 6 and 9, respectively, and light chain CDRs (CDRL1-3) comprising the amino acid sequences of SEQ ID NO: 12, 15 and 18, respectively.

The present invention also relates to an isolated antibody, or antigen-binding fragment thereof, wherein the antibody or fragment comprises one of the following complementary determining region (CDR) sets:
a) heavy chain CDRs 1-3 (CDRH1-3) comprising the amino acid sequences of SEQ ID NO: 1, 4 and 7, respectively, or functional variants thereof having one or two amino acid substitutions with respect to such amino acid sequences and light chain CDRs (CDRL1-3) comprising the amino acid sequences of SEQ ID NO: 10, 13 and 16, respectively, or functional variants thereof having one or two amino acid substitutions with respect to such amino acid sequences;
b) heavy chain CDRs 1-3 (CDRH1-3) comprising the amino acid sequences of SEQ ID NO: 2, 5 and 8, respectively, or functional variants thereof having one or two amino acid substitutions with respect to such amino acid sequences and light chain CDRs (CDRL1-3) comprising the amino acid sequences of SEQ ID NO: 11, 14 and 17, respectively, or functional variants thereof having one or two amino acid substitutions with respect to such amino acid sequences; or
c) heavy chain CDRs 1-3 (CDRH1-3) comprising the amino acid sequences of SEQ ID NO: 3, 6 and 9, respectively, or functional variants thereof having one or two amino acid substitutions with respect to such amino acid sequences and light chain CDRs (CDRL1-3) comprising the amino acid sequences of SEQ ID NO: 12, 15 and 18, respectively, or functional variants thereof having one or two amino acid substitutions with respect to such amino acid sequences.

In particular, the present invention relates to antibodies or antibody fragments comprising a variable domain, heavy chain region (VH) and a variable domain, light chain region (VL), wherein VH comprises complementarity determining regions CDRH1, CDRH2, and CDRH3, wherein VL comprises complementarity determining regions CDRL1, CDRL2, and CDRL3, and wherein (a) an amino acid sequence of CDRH1 is as set forth in any one of SEQ ID NOs: 1-3; (b) an amino acid sequence of CDRH2 is as set forth in any one of SEQ

ID NOs: 4-6; (c) an amino acid sequence of CDRH3 is as set forth in any one of SEQ ID NOs: 7-9; (d) an amino acid sequence of CDRL1 is as set forth in any one of SEQ ID NOs: 10-12; (e) an amino acid sequence of CDRL2 is as set forth in any one of SEQ ID NOs: 13-15; and (f) an amino acid sequence of CDRL3 is as set forth in any one of SEQ ID NOs: 16-18.

The present invention more specifically relates to antibodies or antibodies fragments, wherein:
- (a) an amino acid sequence of CDRH1 is as set forth in SEQ ID NO: 1; (b) an amino acid sequence of CDRH2 is as set forth in SEQ ID NO: 4; (c) an amino acid sequence of CDRH3 is as set forth in SEQ ID NO: 7; (d) an amino acid sequence of CDRL1 is as set forth in SEQ ID NO: 10; (e) an amino acid sequence of CDRL2 is as set forth in SEQ ID NO: 13; and (f) an amino acid sequence of CDRL3 is as set forth in SEQ ID NO: 16;
- (a) an amino acid sequence of CDRH1 is as set forth in SEQ ID NO: 2; (b) an amino acid sequence of CDRH2 is as set forth in SEQ ID NO: 5; (c) an amino acid sequence of CDRH3 is as set forth in SEQ ID NO: 8; (d) an amino acid sequence of CDRL1 is as set forth in SEQ ID NO: 11; (e) an amino acid sequence of CDRL2 is as set forth in SEQ ID NO: 14; and (f) an amino acid sequence of CDRL3 is as set forth in SEQ ID NO: 17; and
- (a) an amino acid sequence of CDRH1 is as set forth in SEQ ID NO: 3; (b) an amino acid sequence of CDRH2 is as set forth in SEQ ID NO: 6; (c) an amino acid sequence of CDRH3 is as set forth in SEQ ID NO: 9; (d) an amino acid sequence of CDRL1 is as set forth in SEQ ID NO: 12; (e) an amino acid sequence of CDRL2 is as set forth in SEQ ID NO: 15; and (f) an amino acid sequence of CDRL3 is as set forth in SEQ ID NO: 18.

The antibodies or antibody fragments of the present invention may be such that they bind to canine, feline and/or equine alpha subunit of the IL-4/IL-13 receptor (IL-4R). This subunit is a part of the receptor complex for the IL-4 and IL-13. These two cytokines are involved in immune regulation and inflammation.

The present invention also relates to an isolated antibody, or antigen-binding fragment thereof, wherein the antibody or fragment comprises one of the following heavy chain (VH) and/or light chain (VL):
a) a VH comprising an amino acid sequence as set forth in SEQ ID NO: 19 and/or a VL comprising an amino acid sequence as set forth in SEQ ID NO: 22;
b) a VH comprising an amino acid sequence as set forth in SEQ ID NO: 20 and/or a VL comprising an amino acid sequence as set forth in SEQ ID NO: 23; or
c) a VH comprising an amino acid sequence as set forth in SEQ ID NO: 21 and/or a VL comprising an amino acid sequence as set forth in SEQ ID NO: 24.

The present invention also relates to an isolated antibody, or antigen-binding fragment thereof, wherein the antibody or fragment comprises one of the following heavy chain (VH) and light chain (VL) sets:
d) a VH comprising an amino acid sequence as set forth in SEQ ID NO: 19 and a VL comprising an amino acid sequence as set forth in SEQ ID NO: 22;
e) a VH comprising an amino acid sequence as set forth in SEQ ID NO: 20 and a VL comprising an amino acid sequence as set forth in SEQ ID NO: 23; or
f) a VH comprising an amino acid sequence as set forth in SEQ ID NO: 21 and a VL comprising an amino acid sequence as set forth in SEQ ID NO: 24.

The present invention also relates to an isolated antibody, or antigen-binding fragment thereof, wherein the antibody or fragment comprises one of the following heavy chain (VH) and light chain (VL) sets:
a) a VH comprising an amino acid sequence at least 90%, at least 95% identical or 100% identical to SEQ ID NO: 19 and a VL comprising an amino acid sequence at least 90%, at least 95% identical or 100% identical to SEQ ID NO: 22;
b) a VH comprising an amino acid sequence at least 90%, at least 95% identical or 100% identical to SEQ ID NO: 20 and a VL comprising an amino acid sequence at least 90%, at least 95% identical or 100% identical to SEQ ID NO: 23; or
c) a VH comprising an amino acid sequence at least 90%, at least 95% identical or 100% identical to SEQ ID NO: 21 and a VL comprising an amino acid sequence at least 90%, at least 95% identical or 100% identical to SEQ ID NO: 24.

In particular, the present invention relates to antibodies or antibody fragments comprising a variable domain, heavy chain region (VH) and a variable domain, light chain region (VL), wherein the VH comprises an amino acid sequence at least about 90% identical to a sequence as set forth in any one of SEQ ID NOs: 19-21, and wherein the VL comprises an amino acid sequence at least about 90% identical to a sequence as set forth in any one of SEQ ID NOs: 22-24. In some instances, the antibodies or antibody fragments comprise VH comprising at least or about 70%, 80%, 85%, 90%, 91%, 92%, 93%, 94%, 95%, 96%, 97%, 98%, 99%, or 100% sequence identity to any one of SEQ ID NOs: 19-21, and VL comprising at least or about 70%, 80%, 85%, 90%, 91%, 92%, 93%, 94%, 95%, 96%, 97%, 98%, 99%, or 100% sequence identity to any one of SEQ ID NOs: 22-24.

In some preferred embodiments, the amino acid sequences of the antibody of the present invention, including CDRs, VHs and/or VLs, have been identified in the canine species. This is advantageous because the sequences match naturally occurring canine antibodies and offer a great degree of compatibility with the canine immune system, and generally reduce immunogenicity.

In some preferred embodiments, the antibody of the present invention is characterized in that it is a fully canine antibody. This means that not only the CDRs originate from canine species, but the entire amino acid sequences-including the variable heavy (VH) and variable light (VL) chains-are also derived from or match those of canine antibodies. This feature provides several advantages, including reduced immunogenicity, enhanced therapeutic efficacy, and improved interaction with canine immune cells, thereby optimizing the effectiveness of the antibodies of the present invention as a treatment for canine diseases.

The present invention also relates to canine antibodies, which are antibodies that are 100% derived from canine immune systems. This means they are fully composed of sequences that are naturally found in canine species, without any genetic modification or sequence derived from another species. The term "canine" is therefore opposed to the term "caninized" according to which the antibody incorporates some elements from canine species, such as the constant regions or specific framework regions, while retaining other components (e.g., CDRs) from different species, such as human or mouse.

Canine antibodies offer several benefits over caninized antibodies. First, they offer reduced immunogenicity, as they reduce the risks of triggering an immune response against the therapeutic antibody, which can occur when non-canine or caninized antibodies are injected. Second, canine antibodies interact more effectively with the immune cells, generally leading to a more potent therapeutic effect. The reason is that they are naturally compatible with the canine immune system. Third, canine antibodies provide a higher degree of specificity and affinity for canine antigens, as they are derived from a species that naturally encounters these antigens. This intrinsic specificity enhances their ability to target and neutralize disease-causing agents or disease markers in dogs more accurately. Additionally, using canine antibodies can reduce the risk of adverse reactions and improve the safety profile of treatments, as they are less likely to provoke unexpected immune responses or complications.

The antibodies of the present invention are preferably obtained from a naive bank, i.e., consisting of antibodies derived from non-immunized animals. These antibodies are part of the natural immune repertoire of the canine species and represent a baseline response. While naive banks provide a more ethical alternative for antibody sourcing, immune banks are often necessary for generating specific antibodies required for targeted applications. Indeed, it is generally considered that therapeutic antibodies can only be obtained from an immune bank for the reasons that the antibodies need to be highly specific and have a strong affinity for their target antigen. These features are usually achieved through immunization, where the immune system produces a targeted response against a specific antigen. In the present invention however, the antibodies not only specifically bind with the target at the required affinity or efficacy needed for therapeutic applications, but have been obtained from a naive bank, which is more ethical since it does not involve painful procedures like immunization. One of the merits of the invention is to provide antibodies 100% derived from canine immune systems that have been obtained from a naïve bank, i.e., from non-immunized animals.

Another merit of the invention is to provide canine antibodies targeting a receptor, more precisely IL-4/IL-13 receptor (IL-4R), i.e., not a soluble factor. Receptors are embedded in cell membranes and have extracellular portions less exposed than soluble factor that are free-floating with more accessible binding sites, making them easier for antibodies to target. The epitopes on receptors are also less accessible due to the embedding in the cell membrane. Additionally, IL-4R receptors undergo conformational changes upon interaction with their ligands and this structural flexibility makes it challenging to identify antibodies that bind effectively and consistently to them.

Another merit of the invention is to provide canine antibodies targeting an endogenic receptor, more precisely IL-4/IL-13 receptor (IL-4R), i.e., which is naturally present in canine species, is non-immunogenic, meaning that it does not naturally trigger an immune response, in contrast to foreign antigens. This immune tolerance makes it challenging to generate antibody responses.

The present invention also relates to chimeric antibodies, which are antibodies comprising components from two distinct species, in particular feline species. In the context of the present invention, "chimeric antibodies" refer to a chimeric antibody having at least a portion of the heavy chain or a portion of the light chain derived from feline or equine species and including variable region derived from the canine species. The variable regions of the canine species are responsible for the specific binding to the antigen, and the constant regions of the feline or equine species are responsible for mediating the antibody's interactions with the immune system components, such as Fc receptors.

The antibody of the present invention, or antigen-binding fragment thereof, may be a monoclonal antibody or an antigen-binding fragment thereof. The antibody of the present invention is preferably a monoclonal antibody.

Monoclonal antibodies can be produced using various techniques, including hybridoma method, recombinant DNA techniques and phage display technologies, or combinations thereof. For instance, the hybridoma method is described by Kohler and Milstein (1975, Nature 256:495), the recombinant DNA methods are described e.g., in U.S. Pat. No. 4,816,567, and isolated monoclonal antibodies may be isolated from phage libraries as outlined, for example, by McCafferty, et al., (1990, Nature 348:552-554).

The antibody of the present invention, or antigen-binding fragment thereof, may preferably bind to IL-4R. The binding to IL-4R may be determined by Enzyme-Linked Immunosorbent Assay (ELISA) by measuring the interaction between the antibody, or antigen-binding fragment thereof, and an immobilized form of IL-4R using enzyme-linked secondary antibodies and a colorimetric or chemiluminescent substrate. Alternatively, the binding to IL-4R may be determined by Enzyme-Linked Immunosorbent Assay (ELISA) by measuring the competitive interaction between the antibody, or antigen-binding fragment thereof, and dupilumab (human antibody) against an immobilized form of IL-4R, using anti-human secondary antibodies and a colorimetric or chemiluminescent substrate. For example, anti-human antibodies-HRP and TMB substrate can be used.

According to the present invention, the antibody, or antigen-binding fragment thereof, is designed to target canine, feline and/or equine IL-4R, bind to it through the antibody's variable regions, i.e., complementarity-determining regions (CDRs), which have a high affinity for a specific epitope on the canine, feline and/or equine IL-4R. By binding to the receptor, the antibody of the present invention, or antigen-binding fragment thereof, prevents both IL-4 and IL-13 from interacting with the receptor. This blockade inhibits downstream signaling pathways of IL-4 and IL-13.

Monoclonal antibodies directed against human IL-4R, i.e., Dupilumab (Dupixent^{™}), a humanized IgG4 Ab, are commercially available and have been utilized in the context of the present invention to serve as reference controls allowing for the comparative assessment of the antibody of the present invention's efficacy, specificity, and overall performance.

The antibody, or antigen-binding fragment thereof, may preferably reduce binding of IL-4 and/or dupilumab (e.g., DUPIXENT^{®}), as determined by ELISA.

The antibody, or antigen-binding fragment thereof, may preferably compete with IL-4 and/or dupilumab (e.g., DUPIXENT^{®}), in binding to canine, feline and/or equine IL-4R, as determined by ELISA.

### Nucleic acid molecules, expression vectors, host cells

The present invention may involve genetic engineering techniques, such as recombinant DNA technology, to produce or modify antibodies. This can include the use of vectors, plasmids, or synthetic DNA to express these antibodies in various host cells, such as bacteria, yeast, or mammalian cells. Additionally, the invention may utilize nucleic acid-based methods, such as phage display or yeast surface display, to select antibody variants with optimal characteristics. These methods involve libraries of antibody fragments or full-length antibodies displayed on the surface of phages or yeast cells, which are screened for binding to specific antigens.

The present invention relates to isolated nucleic acid that encodes the antibody, or antigen-binding fragment thereof, of the present invention.

The present invention also relates to expression vector comprising such nucleic acid, as well as isolated host cell comprising such nucleic acid or such expression vector, and isolated host cell that expresses the antibody or antigen-binding fragment thereof described herein. The invention also relates to a method of producing an antibody or antigen-binding fragment thereof that binds to IL-4R, comprising incubating the host cell under conditions suitable for producing the antibody or antigen-binding fragment thereof.

### Pharmaceutical composition and veterinary treatment

Purified antibody preparations can be combined with one or more pharmaceutically acceptable carriers or excipients and sterilized through filtration to create a pharmaceutical composition. These antibody preparations or compositions may be administered to canine, feline or equine species suffering from an IL-4R-induced condition, including atopic dermatitis, allergic dermatitis, flea allergy dermatitis, contact dermatitis, asthma, seborrhea, pruritus, psoriasis, scleroderma, eczema and pyoderma, and other hypersensitivity reactions involving the IL-4/IL-13R receptor; more preferably, atopic dermatitis. The antibodies are administered in a therapeutically effective amount to alleviate or manage the symptoms associated with these conditions.

Isolated nucleic acid that encodes the antibody, or antigen-binding fragment thereof, of the present invention may also be combined with one or more pharmaceutically acceptable carriers or excipients and sterilized through filtration to create a pharmaceutical composition. Examples of these acceptable carriers or excipients are lipid nanoparticles (LNPs) and Adeno-associated virus (AAV). Reference is made in particular to the following articles:
- Jung HN, Lee SY, Lee S, Youn H, Im HJ. Lipid nanoparticles for delivery of RNA therapeutics: Current status and the role of in vivo imaging. Theranostics. 2022 Oct 24;12(17):7509-7531;
- Salauddin M, Saha S, Hossain MG, Okuda K, Shimada M. Clinical Application of Adenovirus (AdV): A Comprehensive Review. Viruses. 2024; 16(7):1094; and
- Thran M, Mukherjee J, Pönisch M, Fiedler K, Thess A, Mui BL, Hope MJ, Tam YK, Horscroft N, Heidenreich R, Fotin-Mleczek M, Shoemaker CB, Schlake T. mRNA mediates passive vaccination against infectious agents, toxins, and tumors. EMBO Mol Med. 2017 Oct;9(10):1434-1447. doi: 10.15252/emmm.201707678. PMID: 28794134; PMCID: PMC5623855.

Pharmaceutical compositions can be stored in a lyophilized form. The process for preparing the pharmaceutical composition of the preset invention may therefore include a lyophilization step. The lyophilized composition can then be reformulated, typically as an aqueous solution suitable for parenteral administration, prior to administration to canine, feline or equine species. In other embodiments, the pharmaceutical composition may be administered as a liquid, either directly or with appropriate dilution, particularly when the antibody is highly stable against denaturation by heat and oxidation. It can be stored as an aqueous solution ready for administration. Lyophilized compositions can be reconstituted with sterile water for injection (WFI), and antimicrobial agents, such as bacteriostatic agents like benzyl alcohol, may be included. Thus, the present invention provides pharmaceutical compositions in both solid and liquid forms.

The pH of the pharmaceutical composition may range from about pH 5 to about pH 8 when administered. The compositions of the invention are sterile when used for therapeutic purposes. Sterility can be achieved by any of several means known in the art including filtration through sterile filtration membranes (e.g., 0.2 µm membranes). Sterility may be maintained in the presence or absence of antimicrobial agents.

The pharmaceutically acceptable carrier or pharmaceutical composition may notably comprise L-histidine, sodium chloride, and polysorbate 80.

The invention also provides a method of treating a disease or disorder associated with IL-4R dysfunction or disorder in a canine, feline or equine species in need thereof, the method comprising administering to the canine, feline or equine species a therapeutically effective amount of the antibody or antigen-binding fragment thereof of the present invention or of the pharmaceutical composition of the present invention.

The disease or disorder may be a chronic inflammatory skin condition. For example, such skin conditions may be selected in the group consisting of atopic dermatitis, allergic dermatitis, flea allergy dermatitis, contact dermatitis, asthma, seborrhea, pruritus, psoriasis, scleroderma, eczema and pyoderma, and other hypersensitivity reactions involving the IL-4/IL-13R receptor; more preferably, atopic dermatitis. The therapeutically effective amount of the antibody or antigen-binding fragment thereof is preferably utilized to treat atopic dermatitis in canine, feline or equine species.

Additionally provided are methods of using the antibodies, fragments, and polynucleotides described herein for the detection, diagnosis, and monitoring of IL-4R dysfunction or disorder. Provided herein are methods of determining whether a canine, feline or equine species respond to anti-IL-4/IL-13 antibody treatment. In some embodiments, the detection method comprises contacting the sample (e.g., biological sample) with an antibody, fragment, or polynucleotide and determining whether the extent of binding differs from a reference or comparison sample (e.g., control). Provided herein are methods of determining the binding to IL-4R by Enzyme-Linked Immunosorbent Assay (ELISA), including quantitate ELISA, by measuring the interaction between the antibody, or antigen-binding fragment thereof, and an immobilized form of IL-4R, for example using enzyme-linked secondary antibodies and a colorimetric or chemiluminescent substrate. In some embodiments, the methods can be useful for determining whether an antibody or fragment thereof described herein is an appropriate treatment for a subject canine, feline or equine species.

### ASPECTS OF THE INVENTION

The invention may be according to the following aspects:
Aspect 1. An isolated antibody, or antigen-binding fragment thereof, that binds to canine, feline and/or equine alpha subunit of the IL-4/IL-13 receptor (IL-4R), wherein the antibody or fragment comprises one of the following complementary determining region (CDR) sets:
   a) heavy chain CDRs 1-3 (CDRH1-3) comprising the amino acid sequences of SEQ ID NO: 1, 4 and 7, respectively, and light chain CDRs (CDRL1-3) comprising the amino acid sequences of SEQ ID NO: 10, 13 and 16, respectively;
   b) heavy chain CDRs 1-3 (CDRH1-3) comprising the amino acid sequences of SEQ ID NO: 2, 5 and 8, respectively, and light chain CDRs (CDRL1-3) comprising the amino acid sequences of SEQ ID NO: 11, 14 and 17, respectively; or
   c) heavy chain CDRs 1-3 (CDRH1-3) comprising the amino acid sequences of SEQ ID NO: 3, 6 and 9, respectively, and light chain CDRs (CDRL1-3) comprising the amino acid sequences of SEQ ID NO: 12, 15 and 18, respectively.
Aspect 2. An isolated antibody, or antigen-binding fragment thereof, that binds to canine, feline and/or equine alpha subunit of the IL-4/IL-13 receptor (IL-4Rα), wherein the antibody or fragment comprises one of the following heavy chain (VH) and light chain (VL) sets:
   a) a VH comprising an amino acid sequence at least 90%, at least 95% identical or 100% identical to SEQ ID NO: 19 and a VL comprising an amino acid sequence at least 90%, at least 95% identical or 100% identical to SEQ ID NO: 22;
   b) a VH comprising an amino acid sequence at least 90%, at least 95% identical or 100% identical to SEQ ID NO: 20 and a VL comprising an amino acid sequence at least 90%, at least 95% identical or 100% identical to SEQ ID NO: 23; or
   c) a VH comprising an amino acid sequence at least 90%, at least 95% identical or 100% identical to SEQ ID NO: 21 and a VL comprising an amino acid sequence at least 90%, at least 95% identical or 100% identical to SEQ ID NO: 24.
Aspect 3. The antibody or fragment of aspect 1 or 2, wherein the amino acid sequences have been identified in the canine species, preferably using a naive bank.
Aspect 4. The antibody or fragment of any one of the preceding aspects, wherein the antibody is a fully canine antibody.
Aspect 5. The antibody or fragment of any one of aspects 1-3, wherein the antibody is a chimeric antibody.
Aspect 6. The antibody or fragment of any one of the preceding aspects, that is a monoclonal antibody or an antigen-binding fragment thereof.
Aspect 7. The antibody or fragment of any one of the preceding aspects, wherein the antibody or antigen-binding fragment thereof reduces binding of IL-4 and/or dupilumab.
Aspect 8. The antibody or fragment of any one of the preceding aspects, wherein the antibody or antigen-binding fragment thereof compete with IL-4 and/or dupilumab in binding to canine, feline and/or equine IL-4R.
Aspect 9. An isolated nucleic acid that encodes the antibody, or antigen-binding fragment thereof, of any one of aspects 1-8.
Aspect 10. An expression vector comprising the nucleic acid of aspect 9.
Aspect 11. An isolated host cell comprising the nucleic acid of aspect 9 or the expression vector of aspect 10.
Aspect 12. An isolated host cell that expresses the antibody or antigen-binding fragment thereof of any one of aspects 1-8.
Aspect 13. A pharmaceutical composition comprising the antibody of any one of aspects 1-8 and a pharmaceutically acceptable carrier.
Aspect 14. A pharmaceutical composition comprising the nucleic acid of aspect 9 and a pharmaceutically acceptable carrier.
Aspect 15. A method of producing an antibody or antigen-binding fragment thereof that binds to IL-4R, comprising incubating the host cell of aspect 11 or 12 under conditions suitable for producing the antibody or antigen-binding fragment thereof.
Aspect 16. The method of aspect 15, further comprising isolating the antibody or antigen-binding fragment thereof.
Aspect 17. A method of treating a disease or disorder associated with IL-4R dysfunction in a canine, feline or equine species in need thereof, the method comprising administering to the canine, feline or equine species a therapeutically effective amount of the antibody or antigen-binding fragment thereof of any one of aspects 1-8 or the pharmaceutical composition of aspects 13 or 14.
Aspect 18. The method of aspect 17, wherein the disease or disorder is a chronic inflammatory skin condition; preferably selected in the group consisting of atopic dermatitis, allergic dermatitis, flea allergy dermatitis, contact dermatitis, asthma, seborrhea, pruritus, psoriasis, scleroderma, eczema and pyoderma, and other hypersensitivity reactions involving the IL-4/IL-13R receptor; more preferably, atopic dermatitis.
Aspect 19. The antibody or antigen-binding fragment thereof of any one of aspects 1-8 for use in a method of treating a disease or disorder associated with IL-4R dysfunction in canine, feline or equine species in need thereof, the method comprising administering to the canine, feline or equine species a therapeutically effective amount of the antibody, or antigen-binding fragment thereof, of any one of aspects 1-8 or a therapeutically effective amount of the nucleic acid of aspect 9.

Although the foregoing invention has been described in some detail by way of illustration and example for purposes of clarity of understanding, one of skill in the art will appreciate that certain changes and modifications may be practiced within the scope of the appended claims. In addition, each reference provided herein is incorporated by reference in its entirety to the same extent as if each reference was individually incorporated by reference. Where a conflict exists between the instant application and a reference provided herein, the instant application shall dominate.

### EXAMPLES

### Example 1. Preparation of antigen dIL-4R and ligand dIL-4 recombinant protein

The objective of this step was to produce recombinant proteins required for the study, i.e., IL-4R as the antigen of interest (called herein "dIL-4R") and cytokine IL-4 as the corresponding ligand (called herein "dIL-4"). These recombinant proteins were employed in phage display techniques to identify antibodies binding to the target IL-4R.

### Gene synthesis and sub-cloning in expression vector

Mammalian cells: cDNA of dIL-4 and dIL-4R with 6 His-tag at 3'/Cter and signal peptide for secretion (SIP) at 5'/Nter were chemically synthesized with codon optimization for mammalian systems, then sub-cloned in expression vector pTXs1 (ProteoGenix).

The full protein sequence of SIP-dIL-4-6His-MC (115 aa) is as follows:

MGWSCIILFLVATATGVHSNFNITIKEIIKMLNILTARNDSCMELTVKDVFTAPKNTSD
KEIFCRAATVLRQIYTHNCSNRYLRGLYRNLSSMANKTCSMNEIKKSTLKDFLERLK
VIMQKKYYRHGSHHHHHH*
(SEQ ID NO:25)

The full protein sequence of SIP-dIL-4R-6His-MC (214 aa) is as follows:

MKHLWFFLLLVAAPRWVLSKVLHEPSCFSDYISTSVCQWKMDHPTNCSAELRLSYQ
LDFMGSENHTCVPENREDSVCVCSMPIDDAVEADVYQLDLWAGQQLLWSGSFQPS
KHVKPRTPGNLTVYPNISHTWLLMWTNPYPTENHLHSELTYMVNVSNDNDPEDFKV
YNVTYMGPTLRLAASTLKSGASYSARVRAWAQTYNSTWSDWSPSTTWLNYYEPWE
QHLGSHHHHHH*
(SEQ ID NO:26)

*E. coli:* cDNA of dIL-4 and dIL-4R with 6 His-tag at 3'/Cter were chemically synthesized with codon optimization for *E. coli,* then sub-cloned in expression vector (ProteoGenix).

The full protein sequence of dIL-4-6His-EC (116 aa) is as follows:

MNFNITIKEIIKMLNILTARNDSCMELTVKDVFTAPKNTSDKEIFCRAATVLRQIYTHN
CSNRYLRGLYRNLSSM
ANKTCSMNEIKKSTLKDFLERLKVIMQKKYYRHGSHHHHHH*
(SEQ ID NO:27)

The full protein sequence of dIL-4R-6His-EC (215 aa) is as follows:

MKVLHEPSCFSDYISTSVCQWKMDHPTNCSAELRLSYQLDFMGSENHTCVPENRED
SVCVCSMPIDDAVEADVYQLDLWAGQQLLWSGSFQPSKHVKPRTPGNLTVYPNISH
TWLLMWTNPYPTENHLHSELTYMVNVSNDNDPEDFKVYNVTYMGPTLRLAASTLK
SGASYSARVRAWAQTYNSTWSDWSPSTTWLNYYEPWEQHLGSHHHHHH*

(SEQ ID NO:28)

### Expression and purification

Mammalian cells: The pTXs1 constructs were transfected in XtenCHO^{™} cells by using a transient transfection protocol (ProteoGenix). Cell culture medium was collected 14-days post-transfection and a purification was conducted using affinity chromatography with Ni-NTA resin to isolate the recombinant proteins.

Two purified proteins **dIL-4-MC** and **dIL-4R-MC** were obtained and selected for ELISA validation and subsequent phage display projects, as they exhibited good solubility and purity, and underwent post-translational modifications similar or close to the native dog protein.

*E. coli:* the constructs were expressed in *E. coli* cells, then purified by affinity against 6 His tag using Nickel resin. The purified protein **dIL-4-EC** was used for competition with dupilumab.

Additionally, the purified proteins **dIL-4R-MC** and **dIL4-R-EC** were used to demonstrate the importance of post-translation modifications such as glycosylation on dIL4R proteins for their ability to bind to their ligands dIL-4. Indeed, proteins expressed in *E. coli* do not undergo glycosylation, while mammalian cells possess the necessary cellular machinery to perform complex glycosylation, including both N-linked and O-linked glycosylation.

dIL-4-Fc proteins were also produced for further testing.

### Example 2. ELISA validation of the binding between the antigen dIL4R and its ligand dIL-4

The objective of this example is to confirm the interaction between the recombinant protein dIL-4R and its ligand dIL-4 through ELISA experiments.

The proteins were first detected through their His-tags. Plates were coated with the His-tag-purified proteins, followed by incubation with anti-His antibodies and 3,3',5,5'-tetramethylbenzidine (TMB) substrate to produce a detectable signal, which was then measured. All proteins produced according to example 1 were detected with anti-His antibodies in ELISA.

Biotinylation of each protein was then performed to enhance detection in ELISA assays. Plates were coated with biotin-purified proteins, followed by incubation with streptavidin-HRP and TMB substrate to produce a detectable signal, which was then measured.

Both proteins were detected using Streptavidin-HRP in direct ELISA.

### ELISA titration with coated dIL-4R - binding of dupilumab

Plates were coated with the dIL-4R proteins, followed by incubation with dupilumab antibodies, then anti-human-HRP and TMB substrate to produce a detectable signal, which was then measured.

**Table 1. ELISA titration with coated dIL-4R-MC - binding of dupilumab**

| **Dupilumab (µg/ml)** | **dIL-4R-MC** | | **dIL-4R-EC** | | **Negative control** | |
|---|---|---|---|---|---|---|
| | Rep 1 | Rep 2 | Rep 1 | Rep 2 | Rep 1 | Rep 2 |
| 100 | 2.99 | 2.87 | 1.47 | 1.39 | 0.02 | 0.02 |
| 75 | 2.86 | 2.82 | 1.16 | 1.12 | 0.01 | 0.02 |
| 50 | 2.96 | 2.90 | 0.46 | 0.39 | 0.02 | 0.02 |
| 10 | 2.80 | 2.82 | 0.27 | 0.34 | 0.02 | 0.01 |
| 5 | 2.37 | 2.45 | 0.32 | 0.35 | 0.03 | 0.02 |
| 2 | 1.91 | 1.83 | 0.41 | 0.31 | 0.02 | 0.02 |
| Blank | 0.02 | 0.01 | 0.02 | 0.02 | 0.02 | 0.01 |

The results in Table 1 demonstrate that dupilumab mAb binds to dIL-4R-MC proteins and dIL-4R-EC proteins, indicating the relevance of both, especially of dIL-4R-MC proteins. The epitope or binding site of dupilumab on IL-4R seems to be conserved between human and dog species. This suggests that the IL-4R protein or its functional domain is sufficiently conserved between human and canine species, making it suitable for use in competition testing.

### ELISA titration with coated dIL-4R- competition between IL-4 protein and dupilumab

For this ELISA, dIL-4R-MC proteins were first incubated with dupilumab to form a complex. Then, increasing concentrations of dIL-4-EC proteins were added. If dIL-4 proteins bind to the same epitope or a nearby epitope as dupilumab, they will compete with dupilumab for binding, leading to a decrease in dupilumab binding as the concentration of dIL-4 increases. This reduction in binding indicates competitive binding between dIL-4 and dupilumab, demonstrating that dIL-4 proteins bind to an overlapping or nearby epitope on the dIL-4R proteins.

Plates were coated with the dIL-4R-MC proteins, followed by incubation with dupilumab antibodies, then with various concentrations of dIL-4-EC (Table 2) proteins. Incubations with anti-human antibodies-HRP and TMB substrate were then carried out to generate a detectable signal, which was then measured.

**Table 2. ELISA titration with coated dIL-4R- competition between IL-4 protein and dupilumab**

| **dIL-4-EC (µg/ml)** | **dIL-4R-MC + dupilumab** | | **Negative control** | |
|---|---|---|---|---|
| | Rep 1 | Rep 2 | Rep 1 | Rep 2 |
| 150 | 1.73 | 1.69 | 0.02 | 0.02 |
| 100 | 1.79 | 1.72 | 0.02 | 0.03 |
| 50 | 1.82 | 1.78 | 0.03 | 0.01 |
| 10 | 2.20 | 2.15 | 0.02 | 0.01 |
| Blank | 3.02 | 2.98 | 0.02 | 0.02 |

The results in Table 2 show a decrease in binding of dupilumab against dIL-4R-MC upon incubation of increasing concentration of dIL-4-EC, showing a competition between dIL-4-EC and dupilumab for their binding to dIL-4R-MC. These results indicate that dupilumab and IL-4-EC are likely to bind to the same or close epitope of dIL-4R-MC.

### ELISA titration with coated dIL-4R-MC - detection of dIL-4-Fc

Plates were coated with the dIL-4R proteins, followed by incubation with various concentrations of dIL-4-Fc. Incubations with anti-human antibodies-HRP and TMB substrate were then carried out to generate a detectable signal, which was then measured.

**Table 3. ELISA titration with coated dIL-4R-MC - detection of dIL-4-Fc**

| **dIL-4-Fc (µg/ml)** | **dIL-4R-MC protein** | | **Negative control** | |
|---|---|---|---|---|
| | Rep 1 | Rep 2 | Rep 1 | Rep 2 |
| 100 | 2.88 | 2.75 | 0.07 | 0.08 |
| 75 | 2.75 | 2.86 | 0.05 | 0.05 |
| 50 | 2.81 | 2.84 | 0.05 | 0.04 |
| 10 | 2.48 | 2.65 | 0.02 | 0.03 |
| 5 | 2.59 | 2.51 | 0.03 | 0.03 |
| 2 | 2.40 | 2.36 | 0.03 | 0.03 |
| 1 | 1.66 | 1.71 | 0.03 | 0.03 |
| 0.5 | 1.42 | 1.47 | 0.03 | 0.02 |
| Blank | 0.03 | 0.04 | 0.03 | 0.02 |

The results in Table 3 demonstrate a strong binding of dIL-4-Fc on dIL-4R-MC (table 3), but not on dIL-4R-EC (results not shown) which demonstrate the importance of post-translation modifications, such as glycosylation, on dIL-4R proteins.

### Example 3. Incubation with phage display library

The recombinant protein dIL-4R and dIL-4 were demonstrated to bind together in ELISA in example 2. They are therefore suitable for use in phage display. The aim of this example is to perform a phage display antibody selection using dIL-4R antigen and to carry out a competitive elution using either dIL-4 ligand or dupilumab antibody. This approach is designed to identify antibodies that specifically bind to the same or overlapping epitopes on the target protein, enabling the isolation of antibodies with the desired neutralizing or blocking activities.

Phage display panning and screenings are employed to identify phage-displayed antibodies that bind to the target protein.

dIL-4R recombinant protein were produced in HEK cells (MedChemExpress) which offer a post-translational modification profile, such as glycosylation, that closely resembles that of native proteins, making it ideal for applications that require biologically active and properly folded proteins. They are called herein **dIL-4R-HEK.**

During the biopanning process, the scFv dog naive LiAb-SFDog^{™} phage library was incubated with the immobilized target protein, resulting in the binding of phages displaying antibodies with an affinity for the target. This approach successfully enriched the pool for phages specifically interacting with the target protein.

After biopanning, the phage library was subjected to several rounds of washing to remove non-binding phages, followed by elution with Glycine-HCl to isolate those with high affinity for the target protein. The eluted phages were added to *E. coli* TG1 to allow for phage infection and replication, then amplified. The phages were then tested by polyclonal phage ELISA, monoclonal phage ELISA, and positive clones validation ELISA to identify those with the desired binding characteristics. This screening process led to the selection of 6 phages displaying antibodies that specifically interact with the target protein.

### Results

**Table 4. Biopanning results**

| **Rounds** | **Coated antigen** | **Elution** | **Phage quantity** | |
|---|---|---|---|---|
| | | | input | output |
| 1 | dIL-4R-HEK (40 µg/ml) | Gly-HCl | 2 × 10¹² | 2.8 × 10⁵ |
| 2 | dIL-4R-HEK (40 µg/ml) | Gly-HCl | 2 × 10¹² | 3.4 × 10⁷ |
| 3a | dIL-4R-HEK (40 µg/ml) | competition | 2 × 10¹² | 9.2 × 10⁶ |
| 3b | dIL-4R-HEK (40 µg/ml) | Gly-HCl | 2 × 10¹² | 6.2 × 10⁷ |

The data indicated significant enrichment of specific phages over the rounds (highlighted in bold). Given the promising enrichment observed in rounds 3a and 3b, the decision was made to halt further rounds to prevent a decrease in phage diversity.

**Table 5. Results of polyclonal phage ELISA**

| **Phage quantity (pfu/well)** | **Round 1** | | | **Round 2** | | | **Round 3a** | | | **Round 3b** | | |
|---|---|---|---|---|---|---|---|---|---|---|---|---|
| | **Ag** | **NC1** | **NC2** | **Ag** | **NC1** | **NC2** | **Ag** | **NC1** | **NC2** | **Ag** | **NC1** | **NC2** |
| 1 × 10¹² | 0.29 | 0.15 | 0.13 | 3.50 | 0.78 | 0.45 | 4.26 | 1.29 | 0.69 | 4.92 | 2.54 | 1.81 |
| 3.3 × 10¹¹ | 0.13 | 0.10 | 0.06 | 3.69 | 0.63 | 0.26 | 3.91 | 0.91 | 0.58 | 4.95 | 2.09 | 1.33 |
| 1.1 × 10¹¹ | 0.07 | 0.08 | 0.03 | 3.38 | 0.23 | 0.18 | 3.71 | 0.75 | 0.42 | 4.82 | 1.27 | 1.08 |
| 3.6 × 10¹⁰ | 0.05 | 0.04 | 0.03 | 1.74 | 0.09 | 0.07 | 3.46 | 0.46 | 0.29 | 4.10 | 0.79 | 0.53 |
| 1.2 × 10¹⁰ | 0.03 | 0.03 | 0.03 | 0.69 | 0.05 | 0.04 | 3.21 | 0.34 | 0.15 | 3.72 | 0.43 | 0.12 |
| 4 × 10⁹ | 0.03 | 0.03 | 0.02 | 0.23 | 0.03 | 0.03 | 3.28 | 0.12 | 0.09 | 3.94 | 0.26 | 0.18 |
| 1.3 × 10⁹ | 0.03 | 0.03 | 0.02 | 0.10 | 0.03 | 0.02 | 3.28 | 0.05 | 0.04 | 3.39 | 0.09 | 0.10 |
| 0 | 0.03 | 0.03 | 0.02 | 0.02 | 0.02 | 0.02 | 0.02 | 0.02 | 0.02 | 0.02 | 0.02 | 0.03 |

Ag: coated with antigen, dIL-4R-HEK, 4 µg/ml
NC1: coated with non-related protein with HisTag
NC2: coated with buffer

The data confirmed significant enrichment of phages with high binding to the antigen (bold) and lower background signal in rounds 2 and 3. Given that the output from round 2 and round 3a exhibited the best enrichment over their control, these rounds were selected for further monoclonal ELISA testing against the antigen. See below.

After screening of a total of 288 clones, 102 positive clones were identified in the monoclonal phage ELISA. To further distinguish which of these clones are competitive, a competitive ELISA was conducted using a mixture of ligands. Each positive clone was tested with incubation with either PBS (control) or with ligand mix (test of competition). Ligand mix is dupilumab and dIL-4 at ratio 1:1.

**Table 6. Results of competitive ELISA of the 6 single phages from Round 2 and 3a - Coating with dIL-4R-HEK (4 µg/ml) and incubation with PBS (control) or with dIL-4-Fc**

| **Clone** | **Control** | **dIL-4-Fc** |
|---|---|---|
| R2P1-B7 | 2.97 | 2.02 |
| R2P1-C11 | 4.52 | 2.63 |
| R3P1-B1 | 4.10 | 2.45 |
| R3P1-D3 | 4.33 | 1.55 |
| R3P1-C4 | 4.05 | 2.36 |
| R3P1-G6 | 4.33 | 0.4 |

The 6 competitive clones identified in table 6 were tested in competitive ELISA using individual ligands.

**Table 7. Competitive ELISA of 2 positive phages identified from Round 2 - Coating with dIL4R-HEK (4µg/ml) and incubation with PBS (control) or with dIL4-Fc**

| **Clone** | **R2P1-B7** | | **R2P1-C11** | |
|---|---|---|---|---|
| **Ligand concentration** (µg/ml) | **Dupilumab** | **dIL-4-Fc** | **Dupilumab** | **dIL-4-Fc** |
| 200 | **1.73** | 3.44 | **1.18** | 4.14 |
| 50 | **1.96** | 3.35 | **1.72** | 4.28 |
| 12.5 | **2.03** | 3.43 | **2.88** | 4.14 |
| 3.125 | **2.62** | 3.26 | **3.77** | 4.19 |
| 0.78125 | 2.75 | 3.33 | 4.03 | 4.06 |
| 0.1953125 | 2.93 | 3.40 | 4.29 | 4.30 |
| 0.048828125 | 3.03 | 3.34 | 4.00 | 4.14 |
| 0 | 3.24 | 3.41 | 4.24 | 4.29 |

**Table 8. Competitive ELISA of 4 positive phages identified from Round 3a - Coating with dIL-4R-HEK (4µg/ml) and incubation with PBS (control) or with dIL-4-Fc**

| **Clone** | **R3P1-B1** | | **R3P1-D3** | | **R3P1-C4** | | **R3P1-G6** | |
|---|---|---|---|---|---|---|---|---|
| **Ligand concentration** (µg/ml) | **Dupilu mab** | **dIL-4-Fc** | **Dupilu mab** | **dIL-4-Fc** | **Dupilu mab** | **dIL-4-Fc** | **Dupilu mab** | **dIL-4-Fc** |
| 200 | **2,55** | 4,54 | **0,22** | **2,81** | **0,15** | **3,02** | **0,13** | **3,09** |
| 50 | **2,90** | 4,35 | **0,39** | **3,08** | **0,34** | **3,44** | **0,26** | **3,48** |
| 12.5 | **3,40** | 4,49 | **1,07** | **3,23** | **1,05** | **3,89** | **1,13** | **3,66** |
| 3.125 | **4,20** | 4,26 | **2,91** | **3,43** | **2,76** | **4,06** | **3,27** | **3,72** |
| 0.78125 | 4,18 | 4,45 | **3,63** | **3,55** | **3,34** | 4,28 | **3,50** | 3,97 |
| 0.1953125 | 4,56 | 4,37 | **3,77** | 3,89 | **3,27** | 4,36 | **3,96** | 4,11 |
| 0.048828125 | 4,20 | 4,34 | **3,88** | 3,99 | **4,11** | 4,32 | **4,09** | 4,15 |
| 0 | 4,47 | 4,51 | 4,20 | 4,18 | 4,35 | 4,53 | 4,33 | 4,37 |

These results confirmed the 6 competitive clones identified in Tables 7 and 8 exhibited positive binding to the antigen dIL-4R(HEK). We observe a lower signal upon incubation of each of the 6 clones with the ligand Dupilumab, and a lower signal upon incubation of 3 clones with the ligand dIL-4-Fc. These results indicate a potential competition between the antibodies identified and the corresponding ligands. The sequences of these 6 binders are available, and the corresponding antibodies can be produced as IgG antibodies.

### Example 4 - selection of three antibodies

Tables 9 and 10 show exemplary sequences for CDRH1-H3 and CDRL1-L3 as well as variant heavy chains and variant light chains for the three antibodies of the present invention, i.e., clones R3P1-G6, R3P1-D3, R3P1-C4.

**Table 9. CDR sequences**

| **Variable Heavy Chain CDR1 (CDRH1)** | | |
|---|---|---|
| **SEQ ID NO:** | **IgG** | **Amino acid Sequence** |
| 1 | R3P1-G6 | GFTFSSYG |
| 2 | R3P1-D3 | GFTFSDYG |
| 3 | R3P1-C4 | GFTFTDYS |

| **Variable Heavy Chain CDR2 (CDRH2)** | | |
|---|---|---|
| **SEQ ID NO:** | **IgG** | **Amino acid Sequence** |
| 4 | R3P1-G6 | IRHDGTVT |
| 5 | R3P1-D3 | ISRSGSIT |
| 6 | R3P1-C4 | INIGGSNT |

| **Variable Heavy Chain CDR3 (CDRH3)** | | |
|---|---|---|
| **SEQ ID NO:** | **IgG** | **Amino acid Sequence** |
| 7 | R3P1-G6 | ANSRFDY |
| 8 | R3P1-D3 | ARGIGTLDY |
| 9 | R3P1-C4 | AAGFSDSLRY |

| **Variable Light Chain CDR1 (CDRL1)** | | |
|---|---|---|
| **SEQ ID NO:** | **IgG** | **Amino acid Sequence** |
| 10 | R3P1-G6 | SSNIGGFN |
| 11 | R3P1-D3 | SEHSNYI |
| 12 | R3P1-C4 | SSNVGYGDY |

| **Variable Light Chain CDR2 (CDRL2)** | | |
|---|---|---|
| **SEQ ID NO:** | **IgG** | **Amino acid Sequence** |
| 13 | R3P1-G6 | NTS |
| 14 | R3P1-D3 | VRSDGSY |
| 15 | R3P1-C4 | DSS |

| **Variable Light Chain CDR3 (CDRL3)** | | |
|---|---|---|
| **SEQ ID NO:** | **IgG** | **Amino acid Sequence** |
| 16 | R3P1-G6 | STWDNSLKVGV |
| 17 | R3P1-D3 | GTDYKISGQYGHV |
| 18 | R3P1-C4 | SSYENSRSNSV |

**Table 10. IL-4R variable chains sequences**

| **Variable Heavy Chain** | | |
|---|---|---|
| **SEQ ID NO:** | **IgG** | **Amino acid Sequence** |
| 19 | R3P1-G6 | EVQLVESGGDLVTPGGSLTLSCVASGFTFSSYGMSWVRQSPGKGLQWVAW IRHDGTVTYYADAVKGRFTISRDNAKNTLYLQMNSLRAEDTAVYYCANSRF DYWGQGTLVTVSS |
| 20 | R3P1-D3 | GVQLVESGGDLVKPGGSLRLSCVASGFTFSDYGMTWVRQSPGKGLQWVAG ISRSGSITYYTDAVKGRFTISRDNAKNTLYLQMNSLRAEDTAVYYCARGIGT LDYWGQGTLVTISS |
| 21 | R3P1-C4 | EVQLVESGGDLVKPGGSLRLACVASGFTFTDYSMQWVRQAPGKGLQWVA YINIGGSNTNYADTVKGRFTISRDNAKNTLYLQMNSLRAEDTAVYYCAAG FSDSLRYWGQGTLVTVSS |

| **Variable Light Chain** | | |
|---|---|---|
| **SEQ ID NO:** | **IgG** | **Amino acid Sequence** |
| 22 | R3P1-G6 | QAVLNQPASVTGSLGQRVTISCTGSSSNIGGFNVGWFQQFPGTGPRTVIYNT SNRPSGVSDRFSGSKSGSTATLTISGLQAEDEADYYCSTWDNSLKVGVFGG GTHLTVL |
| 23 | R3P1-D3 | LPVLTQPTNASASLEESVKLTCTLSSEHSNYIVQWYQQQPGKAPRYLMYVR SDGSYKRGDGIPSRFSGSSSGADRYLTISNIKSEDEDDYYYCGTDYKISGQY GHVFGSGTHLTVL |
| 24 | R3P1-C4 | QAVLTQPASVSGSLGQRVTISCTGSSSNVGYGDYVGWYQQLPGTSPRTLIF DSSRRPSGVPDRFSGSRSGTTATLTISGLQAEDEADYYCSSYENSRSNSVFG GGTQLTVL |

## Claims

1. An isolated antibody, or antigen-binding fragment thereof, that binds to canine, feline and/or equine alpha subunit of the IL-4/IL-13 receptor (IL-4R), wherein the antibody or fragment comprises one of the following complementary determining region (CDR) sets:
a) heavy chain CDRs 1-3 (CDRH1-3) comprising the amino acid sequences of SEQ ID NO: 1, 4 and 7, respectively, and light chain CDRs (CDRL1-3) comprising the amino acid sequences of SEQ ID NO: 10, 13 and 16, respectively;
b) heavy chain CDRs 1-3 (CDRH1-3) comprising the amino acid sequences of SEQ ID NO: 2, 5 and 8, respectively, and light chain CDRs (CDRL1-3) comprising the amino acid sequences of SEQ ID NO: 11, 14 and 17, respectively; or
c) heavy chain CDRs 1-3 (CDRH1-3) comprising the amino acid sequences of SEQ ID NO: 3, 6 and 9, respectively, and light chain CDRs (CDRL1-3) comprising the amino acid sequences of SEQ ID NO: 12, 15 and 18, respectively.

2. An isolated antibody, or antigen-binding fragment thereof, that binds to canine, feline and/or equine alpha subunit of the IL-4/IL-13 receptor (IL-4Rα), wherein the antibody or fragment comprises one of the following heavy chain (VH) and light chain (VL) sets:
a) a VH comprising an amino acid sequence at least 90%, at least 95% identical or 100% identical to SEQ ID NO: 19 and a VL comprising an amino acid sequence at least 90%, at least 95% identical or 100% identical to SEQ ID NO: 22;
b) a VH comprising an amino acid sequence at least 90%, at least 95% identical or 100% identical to SEQ ID NO: 20 and a VL comprising an amino acid sequence at least 90%, at least 95% identical or 100% identical to SEQ ID NO: 23; or
c) a VH comprising an amino acid sequence at least 90%, at least 95% identical or 100% identical to SEQ ID NO: 21 and a VL comprising an amino acid sequence at least 90%, at least 95% identical or 100% identical to SEQ ID NO: 24.

3. The antibody or fragment of claim 1 or 2, wherein the amino acid sequences have been identified in the canine species using a naive bank.

4. The antibody or fragment of any one of the preceding claims, wherein the antibody is a fully canine antibody.

5. The antibody or fragment of any one of the preceding claims, that is a monoclonal antibody or an antigen-binding fragment thereof.

6. The antibody or fragment of any one of the preceding claims, wherein the antibody or antigen-binding fragment thereof reduces binding of IL-4 and/or dupilumab.

7. The antibody or fragment of any one of the preceding claims, wherein the antibody or antigen-binding fragment thereof compete with IL-4 and/or dupilumab in binding to canine, feline and/or equine IL-4R.

8. An isolated nucleic acid that encodes the antibody, or antigen-binding fragment thereof, of any one of claims 1-7.

9. An expression vector comprising the nucleic acid of claim 8.

10. An isolated host cell comprising the nucleic acid of claim 8 or the expression vector of claim 9.

11. An isolated host cell that expresses the antibody or antigen-binding fragment thereof of any one of claims 1-7.

12. A pharmaceutical composition comprising the antibody of any one of claims 1-7 and a pharmaceutically acceptable carrier.

13. A pharmaceutical composition comprising the nucleic acid of claim 8 and a pharmaceutically acceptable carrier.

14. A method of producing an antibody or antigen-binding fragment thereof that binds to IL-4R, comprising incubating the host cell of claim 10 or 11 under conditions suitable for producing the antibody or antigen-binding fragment thereof.

15. The method of claim 14, further comprising isolating the antibody or antigen-binding fragment thereof.
